# EUROPÄISCHE PATENTSCHRIFT

(11) **EP 1 666 169 B1**
(45) Veröffentlichungstag und Bekanntmachung des Hinweises auf die Patenterteilung: **27.02.2008**
(21) Anmeldenummer: 05023211.5
(22) Anmeldetag: 25.10.2005
(51) Int. Cl.: B09B 3/00, B03B 9/06

(54) **Vorrichtung zur Vor-Ort-Entsorgung von Hygienemüll, insbesondere von Inkontinenz-Artikeln und Verfahren hierfür**
Method and device for the in situ disposal of hygiene waste, especially incontinence articles
Dispositif et procédé d'élimination in situ de déchets d'hygiène, en particulier d'articles d'incontinence

(30) Priorität: 02.12.2004 DE 202004018714 U; 06.01.2005 DE 202005000148 U; 31.01.2005 DE 202005001569 U
(43) Veröffentlichungstag der Anmeldung: 07.06.2006
(73) Patentinhaber: Teeuwen Präzisions GmbH, 41748 Viersen (DE)
(72) Erfinder: Heidel, Werner, 41479 Viersen (DE)
(74) Vertreter: Richter, Werdermann, Gerbaulet & Hofmann

(56) Entgegenhaltungen:
- WO-A-03/009942
- US-A- 5 232 584

## Beschreibung

Die Erfindung betrifft eine Vorrichtung zur Vor-Ort-Entsorgung von Hygienemüll gemäß dem Oberbegriff des Anspruches 1 sowie ein Verfahren hierfür.

Durch die DE 691 24 107D ist ein Verfahren zur Behandlung von absorbierenden sanitären Papierprodukten zur Auftrennung derartiger Produkte in ihre Komponenten bzw. in Komponenten davon bekannt, die eine Form aufweisen, welche zum Rezyklieren bzw. zur Wiederverwendung geeignet ist, wobei die Produkte zumindest Kunststoff- , Zellulose- und superabsorbierende Polymer-("SAP"-) Feststofffraktionen umfassen, wobei das Verfahren folgende Schritte umfasst: Behandeln der Produkte in zerkleinerter Form in einem Bad einer wässrigen Lösung, um lösliches Material zu lösen und das SAP so zu behandeln, dass dessen Quellen in wässrigen Medien gehemmt, verhindert oder umgekehrt wird und Abtrennen der Lösung von löslichem Material von zumindest einer der Feststofffraktionen.

Die zur Durchführung dieses Verfahrens verwendete Vorrichtung umfasst eine drehbare und kippbare, zylindrische Trommel mit einem ersten und einem zweiten Ende, die eine wässrige Lösung beinhalten bzw. aufnehmen kann und aus einer horizontalen Position in eine gekippte Position kippbar ist, in welcher gekippten Position das zweite Ende tiefer als das erste Ende liegt, wobei das erste Ende zum Einbringen des Produktes in das Innere der Trommel und zum Beinhalten des Produktes darin ausgebildet ist, wobei das zweite Ende einen Auslass zur Abgabe des Produktes aus der Trommel besitzt, ferner Mittel zum Drehen der Trommel in zumindest einer von horizontalen und gekippten Positionen, Mittel zum Kippen der Trommel aus der horizontalen in die gekippte Position, wobei zur Behandlung von absorbierenden, sanitären Papierprodukten, um derartige Produkte in ihre Komponenten bzw. in Komponenten davon, deren Form zum Rezyklieren bzw. zur Wiederverwendung geeignet ist, aufzutrennen und zwar unter Verwendung von Mitteln zum Abtrennen von Feststoffen von Flüssigkeiten in der Trommel, einen im Inneren der Trommel befindlichen Rührer, um ein Kommunizieren des Produktes in der Trommel zu bewirken. Mit diesem Verfahren und der hierfür ausgebildeten Vorrichtung soll erreicht werden, dass absorbierende, sanitäre Papierprodukte mit superabsorbierenden Polymer so behandelt werden können, dass einige oder alle der Komponenten derartiger Produkte in einer Form wiedergewonnen werden können, die das Rezyklieren oder eine andere Verwendung dieser Komponenten erleichtert. Eine Entsorgung ist nicht vorgesehen.

Des weiteren ist durch die DE-A-198 53 520 ein Verfahren und eine Vorrichtung zum Entsorgen von Hygienemüll bekannt. Um Volumen und Gewicht von Hygienemüll zu vermindern und die Belästigung durch üble Gerüche abzustellen, wird hier der Hygienemüll nach mechanischer Zerkleinerung mit einer Hygieneflüssigkeit in Berührung gebracht, welche Wasser mit einem in transportablen Toiletten verwendbaren Sanitärzusatz in solchen Mengen enthält, dass der im Hygienemüll enthaltene Zellstoff mit Gelkörnern zusammen mit den darin aufgenommenen Körperausscheidungen aufgelöst wird. Die gebrauchte Hygieneflüssigkeit wird in den Kanal abgeführt, wobei der verbleibende Rest des zerkleinerten Hygienemülls im wesentlichen aus Kunststoff nach dem Entwässern recycelt wird. Die bei diesem Verfahren verwendeten Sanitärzusätze ermöglichen jedoch keine Aufbereitung der im Hygienemüll u. a. enthaltenen Superabsorber, wie Polymere.

Absorbierende sanitäre Papierprodukte bestehen bekannterweise aus einem Vlies aus einem flüssigkeitsdurchlässigen Material, z. B. aus einer flüssigkeitsdurchlässigen Membran aus einem geeigneten Kunststoff oder aus gewebten Produkten aus Baumwolle, einer flüssigkeitsundurchlässigen hinteren Lage, abbaubaren Kunststofffilmen auf Stärkebasis, Webstoff oder Kautschuk und einem absorbierenden oder adsorbierenden Kern aus im Luftstrom aufgebrachten Zellstoffflusen und/oder aus synthetischen zellulosehaltigen gebundenen oder ungebundenen Polypropylenfilamenten, Hanf oder anderen adsorbierenden Fasermaterialien. Der Kern ist in einer Krepp-Umhüllung aus nassfesten Papierlagen oder einem Material mit ähnlichen Eigenschaften eingeschlossen oder eingewickelt. Die Umhüllung des Kerns kann atmungsaktiv, biologisch abbaubar, geruchshemmend oder auf andere Weise abbaubar oder löslich sein. Der Kern kann auch aus einem Material aus superabsorbierenden Polymer-(SAP-)Material entsprechend der DE-A-691 24 107D bestehen. Windeln und Inkontinenz-Produkte benutzen druckempfindliche Klebstoffe für wiederverschließbare Klebebandlaschen oder ähnliche Verschlussmechanismen. Auch können diese Inkontinenz-Produkte mit druckempfindlichen Klebstoffen für Klebstofflinien versehen sein, um eine Befestigung z. B. in Form einer Einlage an der Unterwäsche der Trägerin bzw. Trägers befestigen zu können. Die Verwendung von absorbierenden, sanitären Papierprodukten ist zwar praktisch oder auch notwendig; ihre Entsorgung führt jedoch zu zahlreichen Problemen. Nach den bekannten Verfahren und mittels der bekannten Vorrichtungen können derart absorbierende sanitäre Papierprodukte so behandelt werden, dass einige oder alle der Komponenten derartige Produkte in einer Form wiedergewonnen werden können, die das Recyceln oder eine andere Verwendung dieser Komponenten erleichtert.

Des weiteren ist durch die WO 03/009942 gemäß dem Oberbegriff des Anspruches 1 eine Vorrichtung zur Vor-Ort-Entsorgung von Inkontinenz-Artikeln bekannt. Diese Vorrichtung umfasst ein Grundgehäuse, in dem eine Einrichtung aus einem trommelartigen Gehäuse mit einer oberen Einfüllöffnung und einer im Innenraum des Gehäuses angeordneten, feststehenden oder um eine horizontale Drehachse in Umlauf versetzbare zylindrische Trommel und mit zwei die beiden Trommelöffnungen stirnseitig verschließenden Wandplatten, von denen die erste Wandplatte mit dem Trommelgehäuse verbunden ist und die zweite Wandplatte in Trommeldrehachsenlängsrichtung verschiebbar und um ihre Mittelachse drehbar ausgebildet ist, wobei die beiden stirnseitigen Trommelwandplatten an ihren einander gegenüberliegenden Wandflächen in den Trommelinnenraum hineinreichende Zerkleinerungsmesser tragen, angeordnet ist. Außerdem sind Zuläufe für Waschwasser, für eine Hygieneflüssigkeit und für Chemikalien sowie Ableitungen für die abgesogenen festen Bestandteile des Entsorgungsgutes vorgesehen.

Mit einer derartigen Vorrichtung ist es möglich, Inkontinenz-Artikel vor Ort zu entsorgen mit der Möglichkeit, die einzelnen erhaltenen Komponenten abzuleiten oder zu pelletieren. Dadurch, dass während des Zerkleinerungsprozesses des Entsorgungsgutes der Innenraum der Trommel der Vorrichtung in seiner Größe in einem vorgegebenen Zyklus vergrößert und verkleinert wird, wird auf das Entsorgungsgut im Innenraum der Trommel ein Pressdruck erzeugt, der das Zerkleinern des Entsorgungsgutes vermittels der gegeneinander arbeitenden Messer an den stirnseitigen Wandplatten der Trommel unterstützt wird. Nach dem Entfernen des Waschwassers mit den in diesen gelösten Bestandteilen des Entsorgungsgutes verbleibt zunächst im Innenraum der Trommel die zerkleinerte Feststoffkomponente des Entsorgungsgutes, die ebenfalls vermittels einer staubsaugerartigen Einrichtung aus dem Innenraum der Trommel abgesogen wird. An der Innenwandfläche der Trommel verbleiben dann an der Wandfläche haftende Feststoffteilchen der Entsorgungskomponente, die durch Abziehen der Luft aus dem Innenraum der Trommel durch die lochartigen Durchbrechungen in der Trommelwand entfernt werden. Durch das Absaugen der Luft aus dem Innenraum der Trommel wird ein so genannter Losreißeffekt für die an der Trommelwandfläche haftenden Kunststoffteilchen des Entsorgungsgutes erzeugt. Auch hier wird durch wechselweises Ändern der Größe des Innenraumes der Trommel die Luftdurchtrittsgeschwindigkeit durch die lochartigen Durchbrechungen in der Trommelwand gesteuert, so dass bei einem kleinen Innenraum eine hohe Luftdurchtrittsgeschwindigkeit bei konstantem Volumenstrom erreicht wird. Die Kunststoffteilchen des Entsorgungsgutes werden mit der Luft von der staubsaugerartigen Einrichtung angesogen in dieser getrennt, wobei die hier erhaltene Feststoffkomponente des Entsorgungsgutes in einer beutelförmigen Einrichtung aufgefangen und der Entsorgung zugeführt wird.

Bei dieser Vorrichtung erfolgt das Ableiten des in der Trommel aufbereiteten Inkontinenz-Artikels über die bodenseitige Entnahmeöffnung des Trommelgehäuses und über einen trichterförmigen Stutzen, wobei eine Steuerung von Verschlussklappen für die Entnahmeöffnung vorgesehen ist. An diese senkrecht verlaufende Ableiteinrichtung schließt sich eine Verdichterschnecke an, die gleichzeitig Förderfunktion hat und über die die Kunststoffteilchen des Entsorgungsgutes bei gleichzeitiger Vorwärtsbewegung in Richtung zu einem Auffangbehälter verdichtet. Diese Verdichterschnecke hat hier keine gesonderten Ableitungen für durch die Verdichtung freigewordenes Wasser.

Die US 5 232 584 A offenbart ein Reinigungssystem mit einem einzigen mehrstufigen Reaktor zur Desinfizierung, Waschung und Konzentrierung fester Abfälle, einschließlich kontaminierten Bodens, hochkonzentrierter biologischer Feststoffe, gebrauchten Tierstreus und gebrauchter Windeln. Dabei wird Abfall in den Reaktor dieser Vorrichtung eingebracht. Mindestens ein desinfizierendes und chemisches Agens (einschließlich eines kationischen Tensids) wird zusammen mit Wasser in diesen Reaktor eingebracht, um den genannten festen Abfall zu desinfizieren und mit Hilfe eines mechanischen Mischmittels innerhalb des genannten Reaktors zu verarbeiten. Bei Bedarf wird ein desinfizierendes Gas in denselben Reaktor geleitet. Wenn die Zeit zur Zuführung, Desinfektion, Verarbeitung und Mischung abgelaufen ist, wird das Verfahrenswasser abgelassen. Der desinfizierte und verarbeitete, feuchte, feste Abfall wird in ein Ausstoßkammermittel entladen, das restliches Wasser entfernt und der desinfizierte und verarbeitete feste Abfall kompaktiert.

Das abgelassene Verfahrenswasser und das entfernte Restwasser werden entweder zur Beseitigung in ein städtisches Kanalisationsnetz entsorgt oder in ein chemisches Neutralisierungsmittel zur Vorbehandlung vor der Einleitung in die Kanalisation gebracht oder zur Herstellung eines Stickstoff und Phosphor enthaltenden Düngehilfsstoffes verwendet. Das bei dieser Vorrichtung eingesetzte Ausstoßkammermittel ist ein wellenloser Förderer mit Spiralen, wodurch das Problem des Zersetzens vermieden wird. Bei Betrieb dreht sich der Schneckenförderer im Uhrzeigersinn, wobei er den behandelten festen Abfall zu einem Ende fördert und dabei das ursprünglich im behandelten festen Abfall enthaltene Restwasser durch die Wasserabflüsse ausdrückt und das ausgedrückte Wasser durch einen Wasserauslass abgibt. Anschließend wird der entwässerte feste Abfall durch ein rotierendes Ausstoßmittel an einem Ende abgegeben und der Schneckenförderer beginnt, sich im Gegenuhrzeigersinn zu drehen, wobei er eine neue Charge behandelten Abfalls für ähnliche Kompaktierungs-/Entwässerungs- und Ausstoßvorgänge zu einem entgegengesetzten Ende bringt.

Der vorliegenden Erfindung liegt daher die Aufgabe zugrunde, eine kompakte, kleinsten Raum einnehmende Vorrichtung der eingangs beschriebenen Art von hoher Wirtschaftlichkeit und geringer Störanfälligkeit und mit einer verbesserten Trennung der Feststoffkomponenten von der Waschflüssigkeit zu schaffen, mit der Hygienemüll unter Verwendung eines hohen Schneid-, Zerreiß- und Zerteileffektes in die einzelnen Komponenten zerlegt werden können, um die erhaltenen und zerkleinerten Feststoffkomponenten des Entsorgungsgutes vor Ort zu rezyklieren und somit das Gesamtprodukt wirtschaftlich zu entsorgen oder einer zentralen industriellen Verarbeitung zuzuführen, wobei eine Reduzierung der für die Zerkleinerung und für das Zertrennen des Entsorgungsgutes aufzuwendenden Kräfte reduziert werden sollen. Darüber hinaus soll die Vorrichtung nur wenige Bauteile umfassen, so dass die Vorrichtung wirtschaftlich hergestellt werden kann. Dadurch, dass alle Bauteile auf kleinstem Raum in einem Gehäuse untergebracht sind, ist der Einsatz auch z. B. in Heimen mit geringer Insassenanzahl wirtschaftlich einsetzbar.

Gelöst wird diese Aufgabe mit einer Vorrichtung mit den im Anspruch 1 angegebenen Merkmalen.

Erfindungsgemäß ist die Vorrichtung zur Entsorgung von Hygienemüll gemäß der eingangs beschriebenen Art in der Weise ausgebildet, dass die Ableiteinrichtung als Transportschnecke ausgebildet ist, wobei die Behälterseitenwand bodenseitig in einen konisch sich nach unten verjüngenden Abschnitt übergeht, der in die Transportschnecke mündet, wobei sich an die Transportschnecke die im Innenraum des in etwa senkrecht stehenden, rohrförmigen Gehäuses angeordnete Verdichterschnecke anschließt, wobei die Transportschnecke bis in den bodenseitigen Bereich der Verdichterschnecke geführt ist und wobei die Verdichterschnecke eine unterschiedliche Steigung und eine unterschiedliche Stegstärke aufweist. Die Verdichterschnecke bildet mit einem im Innenraum des Gehäuses angeordneten und sich in Verdichterschneckenlängsrichtung erstreckenden Schersiebblech zum Trennen des zerkleinerten, Feststoffkomponente enthaltende Hygienemülls, von der Waschflüssigkeit mit den in dieser gelösten Bestandteilen des Hygienemülls, eine Funktionseinheit, wobei das Schersiebblech aus einem gewölbtem, plattenförmigen Siebkörper mit einer teilkreisförmigen Wölbung besteht, deren Radius dem Außenradius der Verdichterschnecke entspricht und die gewölbte Siebfläche abstandslos zur Umlauffläche der Verdichterschnecke liegend angeordnet ist, so dass sich ein scherenartiges Zusammenwirken von Verdichterschnecke und Schersiebblech einstellt, wobei eine mit dem Innenraum des Gehäuses mit der Verdichterschnecke verbundene Ableitung mit integrierter Pumpe für die Waschflüssigkeit mit den in dieser gelösten Bestandteilen des Hygienemülls vorgesehen ist, durch die die Waschflüssigkeit einem Abwasserkanal zuführbar ist, wobei im oberen Bereich des Gehäuses mit der Verdichterschnecke der von der Flüssigkeit befreite Rest des zerkleinerten und feste Bestandteile aufweisenden Hygienemülls einem Sammelbehälter zuführbar ist, und wobei im oberen Bereich des Gehäuses mit der Verdichterschnecke eine Verdichterdüse mit einem sich nach oben verjüngenden Abschnitt zum Abtrennen von Restflüssigkeit aus dem Gehäuse ausgebildet ist, die mit der Ableitung verbunden ist.

Mit der erfindungsgemäßen Vorrichtung ist es möglich, Hygienemüll und insbesondere Inkontinenz-Artikel wirtschaftlich vor Ort zu entsorgen mit der Möglichkeit, die einzelnen erhaltenen Komponenten, soweit zulässig, abzuleiten oder gegebenenfalls zu pelletieren. Das Zerkleinern des Entsorgungsgutes erfolgt vermittels der Messerscheibe, ohne dass es hierzu eines hohen Kräfte- bzw. Energieaufwandes bedarf. Die Waschflüssigkeit mit den in dieser gelösten Bestandteilen des Entsorgungsgutes und die zerkleinerten Feststoffkomponenten des Entsorgungsgutes werden aus dem Innenraum des trommelartigen Behälters in eine Verdichterschnecke übergeleitet und mittels einer speziell auf Unterdruck optimierten Wasserpumpe abgezogen, wobei vermittels der Verdichterschnecke in Verbindung mit einem speziell geschaffenen Schersiebbleches dann die Waschflüssigkeit mit den in dieser gelösten Bestandteilen des Entsorgungsgutes von den zerkleinerten Feststoffkomponenten des Entsorgungsgutes getrennt wird. Die Waschflüssigkeit wird dann dem Abwasserkanal zugeführt, wohingegen die zerkleinerten Feststoffkomponenten des Entsorgungsgutes einem Sammelbehälter zugeführt wird, wobei auch die Möglichkeit besteht, vermittels einer geeigneten Einrichtung die Feststoffkomponenten zu pelletieren.

Ein weiterer Vorteil liegt in der Vor-Ort-Entsorgung des aufbereiteten Hygienemülls bzw. der Inkontinenz-Artikel, wobei auch eine stationsweise Entsorgung z. B. in Altenheimen, Krankenhäusern und Kinderheimen möglich ist. Auch ein Pelletieren der Restwertstoffe, wie Kunststoffe und Zellstoffe, ist möglich. Sowohl einzelne als auch größere Mengen an Inkontinenz-Artikeln können entsorgt werden. Der gesamte Prozess kann bei niedrigen Temperaturen durchgeführt werden. Als Waschflüssigkeit wird bevorzugterweise kaltes Waschwasser eingesetzt. Die Bestückung der Vorrichtung kann von Hand oder auch maschinell und automatisch, z. B. vermittels-Förderbänder, vorgenommen werden Vorteilhaft ist, dass eine Entsorgung vor Ort ohne Lagerung und ohne Zwischentransporte möglich ist.

Die Vorrichtung weist nur wenige Bauteile auf, wodurch die Störanfälligkeit der Vorrichtung wesentlich verringert wird. Es hat sich gezeigt, dass mittels der eingesetzten Verdichterschnecke in Verbindung mit dem Schersiebblech eine gute Trennung der Waschflüssigkeit mit den in ihr gelösten Bestandteilen des Entsorgungsgutes von den zerkleinerten Feststoffkomponenten des Entsorgungsgutes erreicht wird, so dass eine zusätzliche Nachbehandlung zur Beseitigung von eventuell noch enthaltender Restflüssigkeit in dem zerkleinerten Entsorgungsgut entfällt.

So ist die Ausbildung der Ableiteinrichtung besonders vorteilhaft, wenn diese Einrichtung als Transportschnecke ausgebildet ist. Die Transportschnecke gewährleistet einen sicheren und zügigen Transport des aus dem Behälterinnenraum abzuleitenden Produktes.

Um eventuell noch vorhandene Restflüssigkeit in dem zerkleinerten Entsorgungsgut zu entfernen, ist vorgesehen, dass im oberen Bereich des Gehäuses mit der Verdichterschnecke der Vorrichtung eine Verdichterdüse mit einem sich nach oben verjüngenden Abschnitt zum Abtrennen von Restflüssigkeit ausgebildet ist, der über eine Verbindungsleitung mit der Ableitung für die Flüssigkeit aus dem Gehäuse mit der Verdichterschnecke verbunden ist.

Vorteilhafte Ausgestaltungen der Erfindung sind Gegenstand der Unteransprüche.

Die Reißmesser der Messerscheibe sind dabei so geformt, dass Hygienemüll bzw. die Inkontinenz-Artikel nicht nur zerschnitten, sondern vor allem aufgerissen und zerhackt werden bei einem Umlauf der Messerscheibe in Arbeitsrichtung. Des weiteren ist die Form, die Ausgestaltung und die Anordnung der Reißmesser so gewählt, dass sich günstige Strömungsverhältnisse bei Betrieb der Vorrichtung einstellen.

Die Erfindung sieht ferner ein Verfahren zur Durchführung einer Vor-Ort-Entsorgung von Hygienemüll unter Verwendung der erfindungsgemäßen Vorrichtung vor, das darin besteht, dass
a.) der Hygienemüll in einem trommelartigen Behälter bei gleichzeitiger Zuführung von Waschwasser, bevorzugterweise kaltem Waschwasser, Chemikalien, Hygieneflüssigkeit und Desinfektionsmitteln vermittels Reißmesser einer umlaufend angetriebenen Messerscheibe aufbereitet, zerrissen und zerkleinert wird, anschließend
b.) das Zerkleinerungsprodukt einer Verdichterschnecke zum Trennen der zerkleinerten, Feststoffkomponenten enthaltenden Hygienemülls von der Waschflüssigkeit vermittels eines Schersiebbleches bei einem gleichzeitigen scherenartigen Zusammenwirken der Verdichterschnecke mit dem Schersiebblech zur Verhinderung eines Verstopfens des Schersiebbleches zugeführt wird, anschließend
c.) die Waschflüssigkeit mit den in dieser gelösten lösbaren Bestandteilen des Hygienemülls einem Abwasserkanal und der von der Waschflüssigkeit befreite Rest des zerkleinerten und feste Bestandteile aufweisenden Hygienemülls einem Sammelbehälter zugeführt wird.

Des weiteren ist vorgesehen, dass für den Aufbereitungsprozess des Hygienemülls in dem Behälter Chemikalien zum Aufbereiten der Superabsorber, wie Feststoffprodukte aus Kunststoff oder superabsorbierend Polymerprodukte oder Gelkörper, bevorzugterweise Calciumchlorid zugeführt werden.

In der Zeichnung ist der Gegenstand der Erfindung beispielsweise dargestellt. Es zeigen:
- Fig. 1: in einer schaubildlichen Ansicht die Entsorgungsvorrichtung mit der mechanischen Zerkleinerung des Hygienemülls bei abgenommenen Wandplatten,
- Fig. 2: schematisch den Aufbau der Entsorgungsvorrichtung mit ihren verschiedenen Bauteilen, wie trommelartigen Behälter mit Messerscheibe, Ableitvorrichtung und Verdichterschnecke mit Schersiebblech,
- Fig. 3: einen senkrechten Schnitt durch einen trommelartigen Behälter,
- Fig. 4: einen senkrechten Schnitt durch den trommelartigen Behälter in der Ausführungsform gemäß Fig. 1,
- Fig. 5: schematisch das Gehäuse mit der Verdichterschnecke und dem Schersiebblech,
- Fig. 6: einen senkrechten Schnitt durch das Gehäuse mit der Verdichterschnecke und dem Schersiebblech gemäß Linie Vl-Vl in Fig. 5,
- Fig. 7: eine schaubildliche Teilansicht des Gehäuses mit der Verdichterschnecke und dem Schersiebblech,
- Fig. 8: die kastenförmige Öffnung des Gehäuses der Verdichterschnecke im Bereich des Schersiebbleches,
- Fig.9: eine schaubildliche Ansicht des Gehäuses mit dem Schersiebblech,
- Fig. 10: eine schaubildliche Seitenansicht eines Reißmessers für die mechanische Zerkleinerung des Hygienemülls,
- Fig. 11: eine Seitenansicht des Reißmessers,
- Fig. 12: eine schaubildliche Rückansicht des Reißmessers,
- Fig. 13: eine Rückansicht des Reißmessers,
- Fig. 14: das Reißmesser in einer Ansicht auf seine Schneidkante und
- Fig. 15: das Reißmesser in einer Ansicht aus seiner Schneid- und Reißkante.

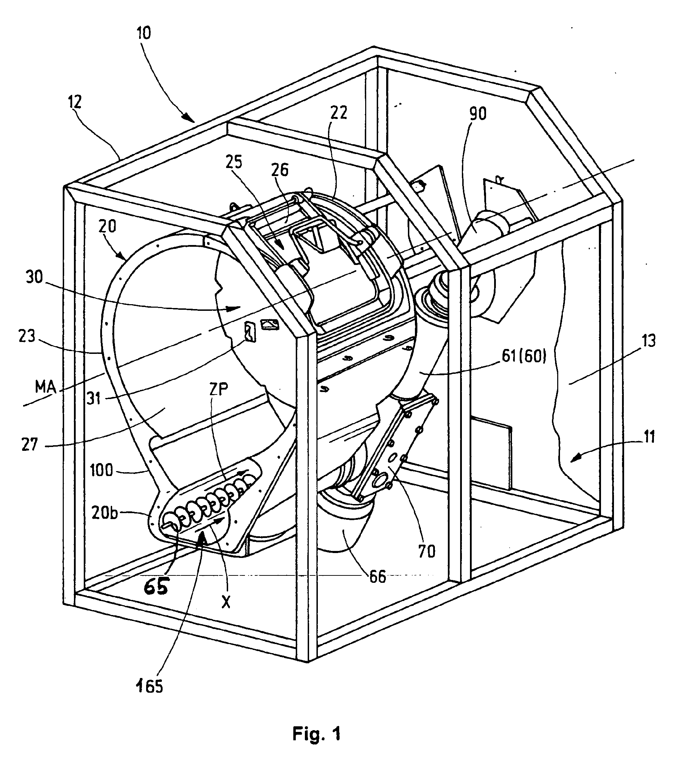

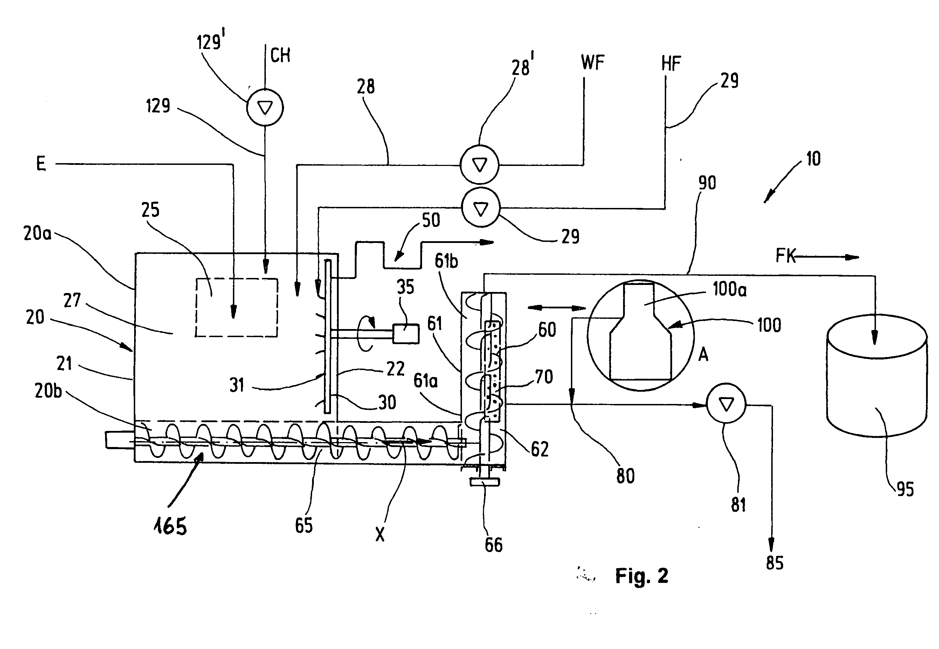

Gemäß Fig. 1 und 2 besteht die Vorrichtung 10 zur Entsorgung von Hygienemüll, insbesondere von Inkontinenz-Artikeln, nachfolgend als Entsorgungsgut E bezeichnet, aus einem Gehäuse 11, das von einem Rahmen 12 mit Seitenwänden 13 gebildet wird.

In dem Rahmen 12 ist ein feststehender, trommelartiger Behälter 20 mit waagerecht verlaufender Mittelachse MA und mit senkrechten Stirnwänden 21, 22 angeordnet. Bevorzugterweise ist der Behälter 20 federnd-elastisch in dem Rahmen 12 gelagert. Der Behälter 20 weist in seinem oberen Bereich eine vermittels eines Deckels 26 verschließbare Einfüllöffnung 25 für das Entsorgungsgut E auf.

Im Innenraum 27 des Behälters 20 ist im Bereich einer seiner beiden Stirnwände 21, 22 eine senkrechte Messerscheibe 30 mit Reißmessern 31 angeordnet, die vermittels einer Antriebseinrichtung 35 umlaufend antreibbar ist, wobei der Umlauf der Messerscheibe 30 konstant in der einen Richtung oder in der anderen Richtung erfolgen kann, wobei bevorzugterweise auch ein ständiger Wechsel der Umlaufrichtung vorgenommen werden kann, so dass die an der Messerscheibe 30 vorgesehenen Reißmesser 31 voll zur Wirkung kommen können und somit der Zerreiß- und Zerkleinerungsprozess des Entsorgungsgutes verbessert wird.

Über Zuleitungen 28, 29, 129 mit integrierten Dosierpumpen bzw. - vorrichtungen 28', 29', 129' werden dem Innenraum 27 des Behälters 20 Waschflüssigkeit WF, bevorzugterweise kaltes Waschwasser, Hygieneflüssigkeit HF zum Unterdrücken von Gerüchen und zur Desinfektion und Chemikalien CH in pulverförmigen oder flüssigen Zustand zugeführt, wobei die Chemikalie zum Aufbereiten der Superabsorber, wie Polymere, eingesetzt werden, die im Hygienemüll und insbesondere in den im Hygienemüll enthaltenen Windeln enthalten sind. Als Chemikalien kommen insbesondere Calciumchloride oder andere geeignete Chemikalien, auch in Kombination mit anderen Chemikalien, zum Einsatz. Eingesetzt werden im wesentlichen solche Chemikalien, die gut entsorgbar sind. Als Desinfektionsmittel können auch Sauerstoff, Ozon, Chlor oder Chlorverbindungen eingesetzt werden.

Für den Aufbereitungsprozess des Hygienemülls werden dem Behälter 20 Chemikalien zum Aufbereiten der Superabsorber, wie Feststoffprodukte aus Kunststoff oder superabsorbierende Polymerprodukte oder Gelkörper, bevorzugterweise Calciumchlorid zugeführt.

Der trommelartige Behälter 20 aus Stahl, Kunststoff oder anderen geeigneten Materialien, weist in seinem Bodenbereich 40 eine in Behälterlängsrichtung verlaufende Ableiteinrichtung 165 auf, die als Transportschnecke 65 ausgebildet ist (Fig. 2). Mit der Transportschnecke 65 wird das sich bodenseitig im Innenraum 27 des Behälters 20 ansammelnde Zerkleinerungsprodukt ZP ausgetragen. Dieses Zerkleinerungsprodukt ZP besteht aus Waschflüssigkeit mit in dieser gelösten Bestandteilen des Entsorgungsgutes E und aus der zerkleinerten Feststoffkomponente FK des Entsorgungsgutes E. Die Ableiteinrichtung 165 kann auch andersartig ausgebildet sein, so z. B. als Transportband, oder als hydraulisch betriebener Schieber. Vermittels dieser Ableiteinrichtung 165 wird das Zerkleinerungsprodukt ZP aus dem Innenraum 27 des Behälters 20 einer Verdichterschnecke 60 in Pfeilrichtung X zugeführt und zwar unter Zuhilfenahme einer Saugpumpe bzw. einer einen Unterdruck aufbauenden Wasserpumpe.

Seitlich des trommelartigen Behälters 20 ist das rohrförmige Gehäuse 61 angeordnet, das die Verdichterschnecke 60 aufnimmt. Das Gehäuse 61 mit der Verdichterschnecke 60 ist senkrecht oder ansteigend geführt (Fig. 1 und 2).

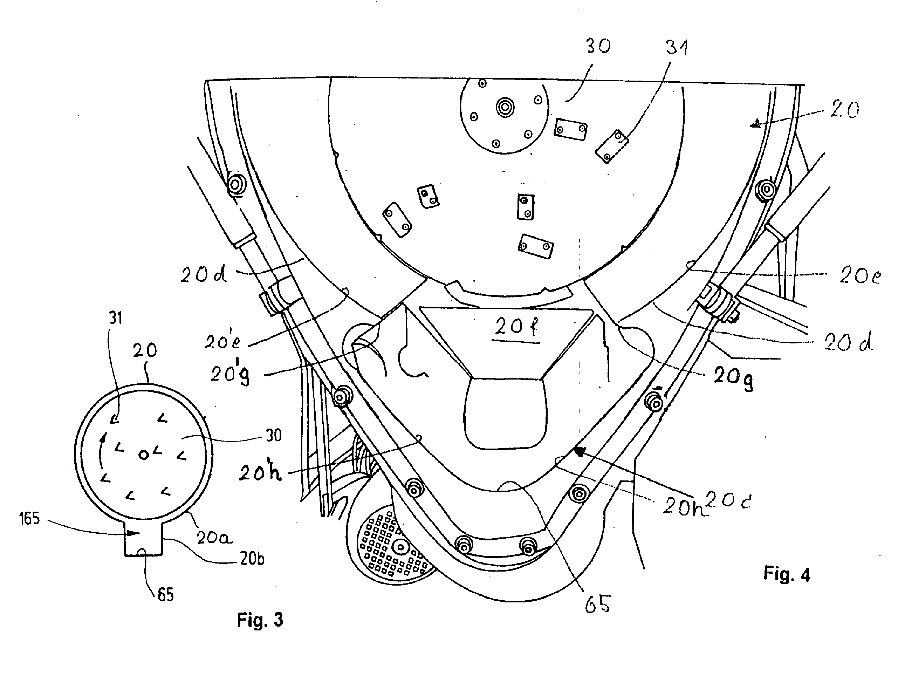

Der trommelartige Behälter 20 besteht aus einem oberen zylindrischen Behälterkörper 20a, der bodenseitig in einen sich verjüngenden Abschnitt 20b übergeht, der die Transportschnecke 65 bildet (Fig. 3). Durch diese Formgebung des Behälters 20 wird während des Betriebes der Vorrichtung eine Verwirbelung des Zerkleinerungsproduktes erreicht, zu der die Form der Reißmesser 31 der Messerscheibe 30 beiträgt.

Fig. 4 zeigt eine Ausführungsform, bei der der trommelartige und zylindrische Behälter 20 bodenseitig in einen konisch sich verjüngenden Abschnitt 20c übergeht, in dessen bodenseitigen Abschluss die Transportschnecke 65 angeordnet ist. Die in den Abschnitt 20c übergehende Trommelwand 20d begrenzt mit ihren Endabschnitten 20e, 20'e eine Öffnung 20f, deren Größe gegenüber dem Trommeldurchmesser kleiner ist. An diese Öffnung 20e schließt sich der Abschnitt 20c mit der Transportschnecke 65 an. Die Behälterwandendabschnitte 20e, 20'e gehen in zungenartige Abschnitte 20g, 20'g über, an die sich die konisch verlaufenden Wandflächen 20f, 20'h des Behälterabschnittes 20c anschließen, so dass die in Fig. 4 dargestellte Querschnittsform des Behälters 20 erhalten wird.

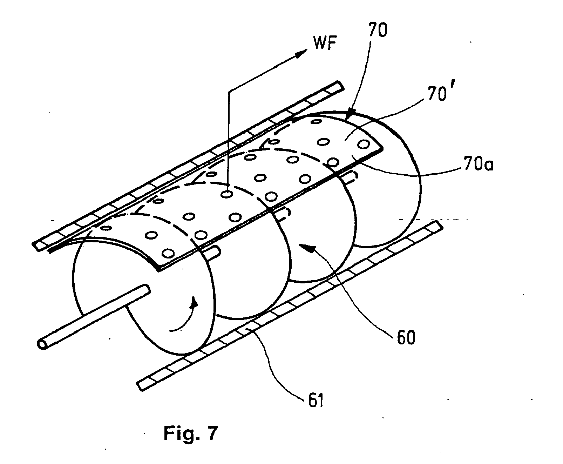

Im unteren Bereich 61a des Gehäuses 61 der Verdichterschnecke 60 steht dessen Gehäuseinnenraum 62 über die Transportschnecke 65 mit dem Innenraum 27 des Behälters 20 in Verbindung, so dass die Transportschnecke 65 bis in den Aufnahmebereich der Verdichterschnecke 60 geführt wird. Der Antrieb für die Transportschnecke 65 ist in den Zeichnungen nicht dargestellt. Auf diese Weise kann das Zerkleinerungsprodukt ZP aus dem Behälter 20 der Verdichterschnecke 60 direkt zugeführt werden. Der Antrieb für die Verdichterschnecke 60 ist bei 66 in Fig. 1 und 2 dargestellt. Als Verdichterschnecke 60 wird eine Ausgestaltung eingesetzt, bei der die Verdichterschnecke unterschiedliche Steigungen und unterschiedliche Stegstärke aufweist. Vermittels der Verdichterschnecke wird das Zerkleinerungsprodukt ZP in den oberen Bereich 61 b des Gehäuses 61 transportiert. Die Abtrennung der Waschflüssigkeit mit den in dieser gelösten Bestandteilen des Entsorgungsgutes E von der zerkleinerten Feststoffkomponente FK des Entsorgungsgutes erfolgt mittels eines in dem Gehäuse 61 vorgesehenen Schersiebbleches 70. Die sich an die Transportschnecke 65 anschließende Verdichterschnecke 60 ist in einem rohrförmigen Gehäuse 61 angeordnet. Die Transportschnecke 65 ist bis in den bodenseitigen Bereich der Verdichterschnecke 60 geführt, die mit dem im Innenraum 62 des Gehäuses 61 angeordneten und sich in Verdichterschneckenlängsrichtung erstreckenden Schersiebblech 70 zum Trennen des zerkleinerten, Feststoffkomponenten enthaltenden Hygienemülls von der Waschflüssigkeit WF mit den in dieser gelösten Bestandteilen des Hygienemülls, eine Funktionseinheit bildet, derart, dass die Verdichterschnecke 60 und das Schersiebblech 70 scherenartig zusammenwirken, um ein Verstopfen des Schersiebbleches 70 zu vermeiden, wobei über eine Ableitung 80 vermittels einer Pumpe 81 die Waschflüssigkeit WF mit den in dieser gelösten Bestandteilen des Hygienemülls abgesogen und einem Abwasserkanal 85 zugeführt wird (Fig. 2, 5, 6 und 7). Im oberen Bereich 61 b des Gehäuses 61 mit der Verdichterschnecke 60 wird der von der Flüssigkeit befreite Rest FK des zerkleinerten und feste Bestandteile aufweisenden Hygienemülls einem Sammelbehälter 95 zugeführt.

Die anfallende Waschflüssigkeit WF mit den in dieser gelösten Bestandteilen des Entsorgungsgutes wird über eine Ableitung 80 mit integrierter Pumpe 81 abgezogen und dem Abwasserkanal 85 zugeführt.

Eine zusätzliche Entfernung von Restflüssigkeit aus dem Zerkleinerungsprodukt ZP kann über eine spezielle Ausgestaltung des Übergangbereiches vom Innenraum 62 des Gehäuses 61 zur Ableitung 80 erreicht werden. Diese Ausgestaltung besteht in einer Verdichterdüse 100 mit einem oberen verjüngenden Abschnitt 100a, die in Fig. 2 bei A dargestellt ist.

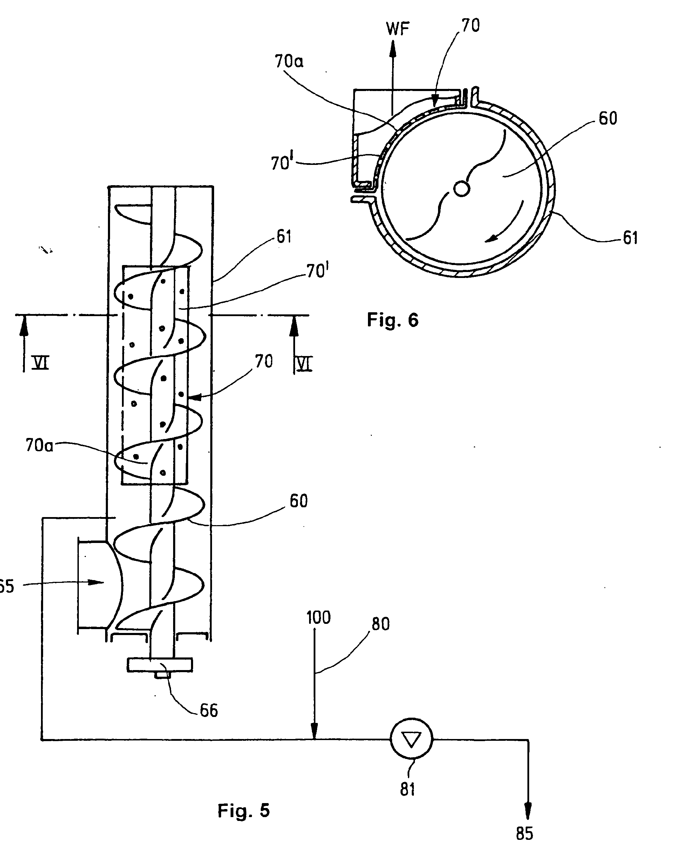

Das Schersiebblech 70 ist als in das Gehäuse 61 der Verdichterschnecke 60 einsetzbarer Einsatzkörper 77 ausgebildet, wobei die gewölbte Siebfläche 70a abstandslos zur Umlauffläche der Verdichterschnecke 60 liegend angeordnet ist, so dass die Verdichterschnecke 60 an der Innenwandfläche des Schersiebbleches 70 entlang schert (Fig. 5, 6 und 7).

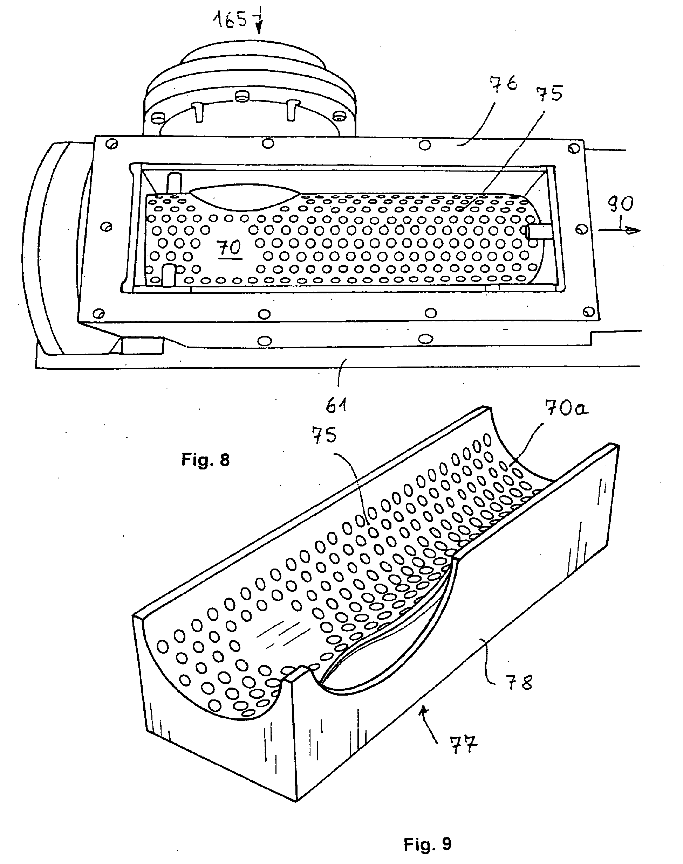

Das Schersiebblech 70 ist, wie in Fig. 8 dargestellt, im Bereich einer in dem die Verdichterschnecke 60 aufnehmenden Gehäuse 61 ausgebildeten, verschließbaren Öffnung 75 in einem kastenförmigen Gehäusestutzen 76 angeordnet, der mit dem Gehäuse 61 verbunden ist. In diesem kastenförmigen Gehäusestutzen 76 ist der als Schersiebblech 70 ausgebildete Einsatzkörper 77 eingesetzt, der aus einem kastenförmigen Rahmen 78 besteht, in dem die gewölbte Siebfläche 70a angeordnet ist (Fig. 9). Der Einsatzkörper 77 ist in dem Gehäuse 61 auswechselbar angeordnet.

Die Messerscheibe 30 trägt auf ihrer dem Innenraum 27 des Behälters 20 zugekehrten Wandfläche eine Anzahl von Reißmessern 31 zum Zerkleinern, Zerteilen und Zerreißen des Entsorgungsgutes. Dabei kann es sich um einzeln angeordnete Reißmesser 31 handeln, jedoch auch ringförmig angeordnete Reißmesser können verwendet werden, wobei neben einem Reißmesserring auch mehrere ineinandergesetzte Reißmesserringe mit sich zum Mittelpunkt verkleinerndem Durchmesser eingesetzt werden können.

Um eine effektive Zerkleinerung, Zerteilung und Zerreißen des Entsorgungsgutes in Verbindung mit der Waschflüssigkeit zu erreichen, ist es vorteilhaft, wenn die Länge des Behälters 20 in etwa dem Durchmesser der Stirnwände 21 bzw. 22 des Behälters 20 entspricht, wobei in Fig. 1 die eine der beiden Stirnwände 21, 22 nicht dargestellt ist. Der Durchmesser der Messerscheibe 30 sollte in etwa dem Durchmesser der Stirnwände 21, 22 im Bereich oberhalb der Transportschnecke 65 des Behälters 20 entsprechen. Der Behälter 20 ist in dem Rahmen 12 des Gehäuses 11 der Vorrichtung 10 feststehend angeordnet und weist eine in etwa zylindrische Form auf.

Um ein einwandfreies Überführen des in dem Behälter 20 aufbereiteten Entsorgungsgutes in die Transportschnecke 65 zu gewährleisten, geht die Behälterseitenwand 23 in einen, bevorzugterweise konisch sich nach unten verjüngenden Abschnitt 20b über, der in die Transportschnecke 65 übergeht (Fig. 1, 2, 3 und 4). Die Transportschnecke 65 verläuft parallel zur Mittellängsachse des Behälters 20.

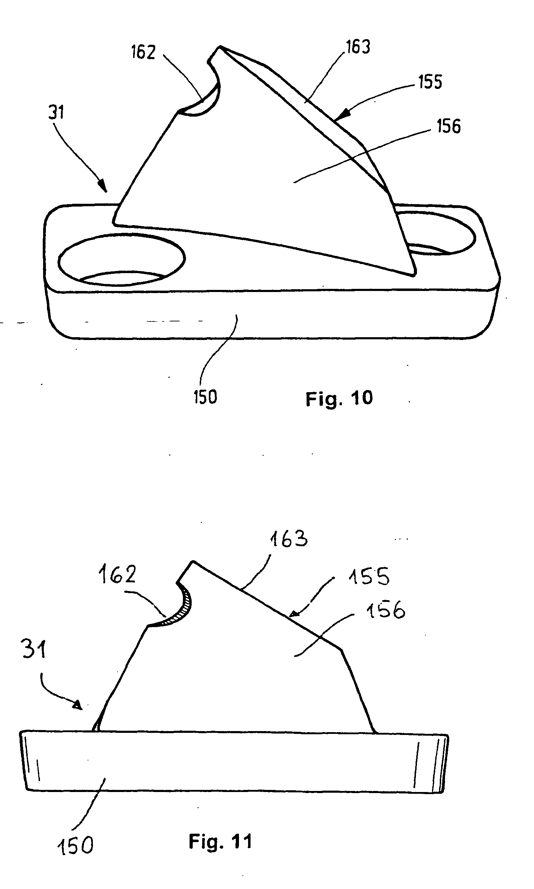

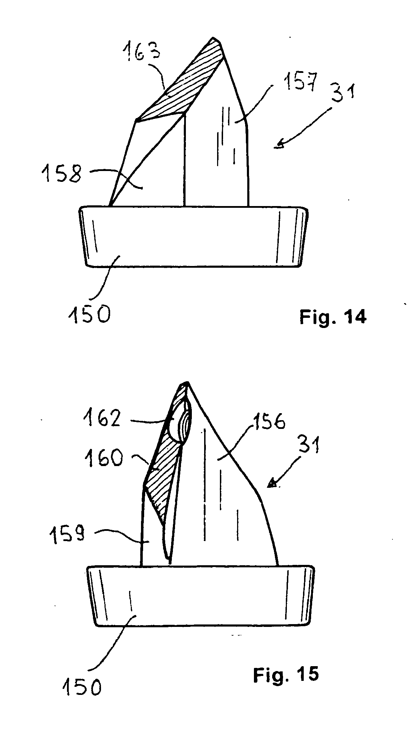

Für eine effektive Zerkleinerung des Hygienemülls weist jedes Reißmesser 31 einen optimierten Messerschliff auf. Gemäß der Ausführungsform gemäß Fig. 10 bis 15 besteht jedes Reißmesser 31 aus einer in etwa rechteckförmigen Befestigungsplatte 150 und aus einem senkrecht auf der Befestigungsplatte 150 stehend angeordneten und diagonal zu dieser verlaufenden platten- und in etwa -dreieckförmigen Messerkörper 155. Die Befestigungsplatte 150 ist mit Durchbrechungen zur Aufnahme von Befestigungsschrauben versehen, um das Reißmesser 31 an der Messerscheibe 30 befestigen zu können, jedoch auch andere Befestigungsarten können zur Anwendung gelangen. Die Befestigungsplatte 150 kann auch eine andere geometrische Formausgestaltung aufweisen, jedoch ist die rechteckförmige Ausgestaltung von Vorteil, da durch die Anordnung des Messerkörpers 155 in diagonaler Richtung zur rechteckförmigen Befestigungsplatte 150 der Messerkörper 155 eine Länge aufweisen kann, die in etwa der Diagonalen der Befestigungsplatte 150 entspricht.

Der Messerkörper 155 ist plattenförmig ausgebildet; er ist senkrecht stehend auf der Befestigungsplatte 150 befestigt und weist in etwa eine dreieckige Formgebung auf, wobei der Messerkörper mit seiner Dreiecksgrundseite auf der Befestigungsplatte 150 aufsitzt.

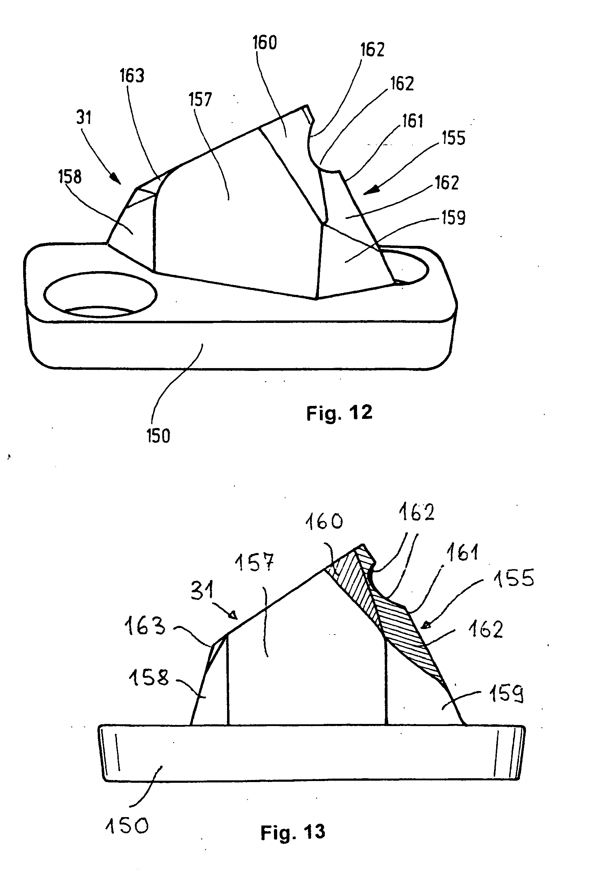

Die eine Seitenwandfläche 156 des Messerkörpers 155 ist bogenförmig nach innen gewölbt (Fig. 10), während die andere Seitenwandfläche 157 plan verlaufend ist (Fig. 11). Diese Seitenwandfläche 157 weist zwei zur Seitenwandfläche abgewinkelte Seitenabschnittsflächen 158, 159 sowie eine im oberen Spitzenbereich des Messerkörpers 155 liegende obere Abschnittsfläche 160 auf, die unter Ausbildung einer Schneidkante 161 mit einer messerartigen eingezogenen Reißkante 162 in eine spitz zulaufende Schneidfläche 162 übergeht (Fig. 12). Die andere sich vom Spitzenbereich des Messerkörpers 155 zur seitlichen Abschnittsfläche 158 erstreckende Seitenkante ist als Schneidkante und Schneidfläche 163 ausgebildet, wobei die eingezogene Reißkante 162 als zweiseitig angeschliffene Einziehung ausgebildet ist.

Aufgrund der speziellen Profilierung des Messerkörpers 155 ist ein Reißmesser 31 geschaffen, das aufgrund verschiedener Flächenanschliffe Schneid- und Reißkanten aufweist, die eine effektive Zerkleinerung des Hygienemülls ermöglichen. Die geometrische Anordnung der Reißmesser 31 auf der Messerscheibe 30 ist so gewählt, dass sich eine optimale Verwirbelung in Arbeits- und Trennrichtung der Messerscheibe 31 ergibt.

Zur Entlüftung ist der Behälter 20 mit einem selbstnachfüllenden Geruchsverschluss 50 versehen (Fig. 2).

Die Steuerung des Zulaufs für die Waschflüssigkeit WF und der Dosierpumpen 28', 29', 129' für die Hygieneflüssigkeit und für die Chemikalie, die Steuerung für die Antriebseinreichung 35 für die Messerscheibe 30 sowie die Steuerungen für die Ableiteinrichtung 165, die Verdichterschnecke 60 und die Pumpen sind in einem Programmschaltwerk zusammengefasst oder werden vermittels einer frei programmierbaren Einrichtung vorgenommen.

## Patentansprüche

1. Vorrichtung zur Vor-Ort-Entsorgung von Hygienemüll, wobei mittels der Vorrichtung der Hygienemüll mechanisch zerkleinerbar und in einer Waschflüssigkeit wenigstens zum Teil derart auflösbar ist, dass die so erhaltene Flüssigkeit vom verbleibenden Rest des zerkleinerten Hygienemülls trennbar, die Flüssigkeit einem Abflusskanal zuführbar und der verbleibende Rest des zerkleinerten Hygienemülls entwässerbar sowie getrennt entsorgbar ist, und wobei die Vorrichtung (10) aus einem Gehäuse (11) besteht, in dem ein feststehender, trommelartiger Behälter (20) mit waagerechter Mittelachse (MA) und mit einer Einfüllöffnung (25) für den Hygienemüll sowie mit einer Zuleitung (28) für eine dosierte Waschflüssigkeit (WF), einer Zuleitung (29) für eine dosierte Hygieneflüssigkeit (HF) zum Unterdrücken von Gerüchen und zur Desinfektion und einer Zuleitung für dosierte Chemikalien in pulvrigem oder flüssigem Zustand zum Aufbereiten der Superabsorber, wobei im Bodenbereich des Behälters (20) eine in den Behälterkörper (20) integrierte Ableiteinrichtung für den Abtransport des zerkleinerten, mit Waschflüssigkeit durchsetzten Hygienemülls aus dem Bodenbereich des Behälters vorgesehen ist, wobei im Innenraum (27) des Behälters (20) im Bereich einer seiner beiden Seitenwände (21; 22) eine umlaufend, antreibbare, senkrechte Messerscheibe (30) mit zum Innenraum (27) des Behälters (20) gerichteten Reismessern (31) zum Aufreißen und Zerkleinern des Hygienemülls angeordnet ist und eine sich an die Ableiteinrichtung anschließende, in einem etwa rohrförmigen Gehäuse angeordnete Verdichterschnecke, wobei die Ableiteinrichtung bis in den bodenseitigen Bereich der Verdichterschnecke geführt ist, angeordnet sind, und wobei ein Programmschaltwerk oder eine frei programmierbare Einrichtung zur Steuerung des Zulaufs der Waschflüssigkeit und der Dosierpumpen (28', 29', 129') für die Hygieneflüssigkeit und für die Chemikalien, der Antriebseinrichtung (35) für die Messerscheibe (30) sowie der Antriebseinrichtung (66) für die Verdichterschnecke (60) und die Pumpen vorgesehen ist,
**dadurch gekennzeichnet,**
**dass**
- die Ableiteinrichtung (165) als Transportschnecke (65) ausgebildet ist, wobei die Behälterseitenwand (23) bodenseitig in einen konisch sich nach unten verjüngenden Abschnitt übergeht, der in die Transportschnecke (65) mündet,
- sich an die Transportschnecke (65) die im Innenraum (62) des in etwa senkrecht stehenden, rohrförmigen Gehäuses (61) angeordnete Verdichterschnecke (60) anschließt, wobei die Transportschnecke (65) bis in den bodenseitigen Bereich der Verdichterschnecke (60) geführt ist, und wobei die Verdichterschnecke (60) eine unterschiedliche Steigung und eine unterschiedliche Stegstärke aufweist,
- die Verdichterschnecke (60) mit einem im Innenraum (62) des Gehäuses (61) angeordneten und sich in Verdichterschneckenlängsrichtung erstreckenden Schersiebblech (70) zum Trennen des zerkleinerten, Feststoffkomponente enthaltende Hygienemülls von der Waschflüssigkeit mit den in dieser gelösten Bestandteilen des Hygienemülls eine Funktionseinheit bildet, wobei das Schersiebblech (70) aus einem gewölbtem, plattenförmigen Siebkörper (70') mit einer teilkreisförmigen Wölbung besteht, deren Radius dem Außenradius der Verdichterschnecke (60) entspricht, und die gewölbte Siebfläche (70a) abstandslos zur Umlauffläche der Verdichterschnecke (60) liegend angeordnet ist, so dass sich ein scherenartiges Zusammenwirken von Verdichterschnecke (60) und Schersiebblech (70) einstellt,
- eine mit dem Innenraum (62) des Gehäuses (61) mit der Verdichterschnecke (60) verbundene Ableitung (80) mit integrierter Pumpe (81) für die Waschflüssigkeit (WF) mit den in dieser gelösten Bestandteilen des Hygienemülls vorgesehen ist, durch die die Waschflüssigkeit (WF) einem Abwasserkanal (85) zuführbar ist, wobei im oberen Bereich (61 b) des Gehäuses (61) mit der Verdichterschnecke (60) der von der Flüssigkeit befreite Rest des zerkleinerten und feste Bestandteile aufweisenden Hygienemülls einem Sammelbehälter (95) zuführbar ist,
- im oberen Bereich (61 b) des Gehäuses (61) mit der Verdichterschnecke (60) eine Verdichterdüse (100) mit einem sich nach oben verjüngenden Abschnitt zum Abtrennen von Restflüssigkeit aus dem Gehäuse (61) ausgebildet ist, die mit der Ableitung (80) verbunden ist.

2. Vorrichtung nach Anspruch 1,
**dadurch gekennzeichnet,**
**dass** der Innenraum (27) des Behälters (20) mit einem Kaltwasserzulauf (28) verbunden ist.

3. Vorrichtung nach einem der Ansprüche 1 und 2,
**dadurch gekennzeichnet,**
**dass** der trommelartige Behälter (20) aus einem oberen zylindrischen Behälterkörper (20a) besteht, der bodenseitig in einen sich verjüngenden Abschnitt (20b) übergeht, der die Transportschnecke (65) bildet.

4. Vorrichtung nach einem der Ansprüche 1 bis 3,
**dadurch gekennzeichnet,**
**dass** das Schersiebblech (70) als in das Gehäuse (61) mit der Verdichterschnecke (60) einsetzbarer Einsatzkörper ausgebildet ist.

5. Vorrichtung nach einem der Ansprüche 1 bis 4,
**dadurch gekennzeichnet,**
**dass** auf der dem Innenraum (27) des Behälters (20) zugekehrten Wandfläche der Messerscheibe (30) eine über die Scheibenfläche verteilt angeordnete Anzahl von Reißmessern (31) vorgesehen sind.

6. Vorrichtung nach einem der Ansprüche 1 bis 5,
**dadurch gekennzeichnet,**
**dass** die Länge des Behälters (20) in etwa dem Durchmesser der kreisförmigen Stirnwände (21, 22) des zylindrischen Behälterkörpers (20a) des Behälters (20) entspricht, wobei der Durchmesser der Messerscheibe (30) in etwa dem Durchmesser der Stirnwände (21, 22) im Bereich oberhalb der Transportschnecke (65) des Behälters (20) entspricht.

7. Vorrichtung nach einem der Ansprüche 1 bis 6,
**dadurch gekennzeichnet,**
**dass** der Behälter (20) in dem Rahmen (12) des Gehäuses (11) federnd-elastisch gelagert ist.

8. Vorrichtung nach einem der Ansprüche 1 bis 7,
**dadurch gekennzeichnet,**
**dass** jedes Reißmesser (31) für eine effektive Zerkleinerung des Hygienemülls einen optimierten Messerschliff aufweist und aus einer in etwa rechteckförmigen Befestigungsplatte (150) und aus einem senkrecht auf der Befestigungsplatte (150) stehend angeordneten und diagonal zu dieser verlaufenden platten- und in etwa dreieckförmigen Messerkörper (155) besteht, dessen eine Seitenwandfläche (156) bogenförmig nach innen gewölbt ist, wobei die andere Seitenwandfläche (157) plan verlaufend ist und zwei zur Seitenwandfläche (157) abgewinkelte Seitenabschnitte (158, 159) sowie eine im oberen Spitzenbereich des Messerkörpers (155) liegende obere abgewinkelte Abschnittsfläche (160) aufweist, die unter Ausbildung einer Schneidkante (161) mit einer messerartigen eingezogenen Reißkante (162) in eine spitz zulaufende Schneidfläche (162) übergeht, wobei die andere vom Spitzenbereich des Messerkörpers (155) zur seitlichen Abschnittsfläche (158) erstreckende Seitenkante als Schneidkante und Schneidfläche (163) ausgebildet ist, wobei die eingezogene Reißkante (162) als zweiseitig angeschliffene Einziehung ausgebildet ist.

9. Vorrichtung nach einem der Ansprüche 1 bis 8,
**dadurch gekennzeichnet,**
**dass** der Behälter (20) zur Entlüftung mit einem selbstnachfüllenden Geruchsverschluss (50) versehen ist.

10. Verfahren zur Durchführung einer Vor-Ort-Entsorgung von Hygienemüll unter Verwendung einer Vorrichtung gemäß den Ansprüchen 1 bis 9,
**dadurch gekennzeichnet,**
**dass**
a.) der Hygienemüll in einem trommelartigen Behälter (20) bei gleichzeitiger Zuführung von Waschwasser, bevorzugterweise kaltem Waschwasser, Chemikalien, Hygieneflüssigkeit und Desinfektionsmitteln vermittels Reißmesser (31) einer umlaufend angetriebenen Messerscheibe (30) aufbereitet, zerrissen und zerkleinert wird, anschließend
b.) das Zerkleinerungsprodukt einer Verdichterschnecke (60) zum Trennen der zerkleinerten, Feststoffkomponenten enthaltenden Hygienemülls von der Waschflüssigkeit vermittels eines Schersiebbleches (70) bei einem gleichzeitigen scherenartigen Zusammenwirken der Verdichterschnecke (60) mit dem Schersiebblech (70) zur Verhinderung eines Verstopfens des Schersiebbleches (70) zugeführt wird, anschließend
c.) die Waschflüssigkeit mit den in dieser gelösten lösbaren Bestandteilen des Hygienemülls einem Abwasserkanal und der von der Waschflüssigkeit befreite Rest des zerkleinerten und feste Bestandteile aufweisenden Hygienemülls einem Sammelbehälter (95) zugeführt wird.

11. Verfahren nach Anspruch 10,
**dadurch gekennzeichnet,**
**dass** für den Aufbereitungsprozess des Hygienemülls in dem Behälter (20) Chemikalien zum Aufbereiten der Superabsorber, wie Feststoffprodukte aus Kunststoff oder superabsorbierende Polymerprodukte oder Gelkörper, zugeführt werden.

## Claims

1. Device for the in situ disposal of hygiene waste, whereby the hygiene waste is mechanically disintegrated by means of the device and is dissolved at least partially in a washing liquid in such a manner that the liquid thus obtained can be separated form the remaining rest of the disintegrated hygiene waste, the liquid can be fed to a discharge duct and the remaining rest of the disintegrated hygiene waste can be drained as well as separately disposed of and whereby the device (10) consists of a housing (11) in which a stationary drum-type container (20) is provided with a horizontal central axis (MA) and with a filling opening (25) for the hygiene waste as well as with a supply duct (28) for a metered washing liquid (WF), a supply duct (29) for a metered sanitary liquid (HF) for suppressing odours and for disinfection and a supply duct for metered chemicals in powdered or liquid state for treating the superabsorbers, whereby a discharge device, integrated in the container body (20), is provided in the bottom area of the container (20) for carrying away the disintegrated hygiene waste mixed with washing liquid from the bottom area of the container, whereby a rotatably drivable vertical knives disk (30) with shearing knives (31) directed to the inner space (27) of the container (20) for tear opening and disintegrating the hygiene waste is placed in the inner space (27) of the container (20) in the area of one of its two side walls (21; 22) and a compressor screw, following the discharge device, placed in an approximately tubular housing, whereby the discharge device is guided into the bottom-sided area of the compressor endless screw and whereby a program switching device or a free programmable device is provided for the control of the admission of the washing liquid and of the metering pumps (28', 29', 129') for the sanitary liquid and for the chemicals, of the driving device (35) for the knives disk (30) as well as of the driving device (66) for the compressor endless screw (60) and the pumps,
**characterized in**
**that**
- the discharge device (165) is configured as a transport screw (65), whereby the container side wall (23) ends on the bottom side into a downwards conically tapering section which ends into the transport screw (65),
- the compressor screw (60) placed in the inner space (62) of the tubular housing (61) approximately vertically standing follows the transport screw (65), whereby the transport screw (65) is guided into the bottom-sided area of the compressor screw (60) and whereby the compressor screw (60) has a different pitch and a different web thickness,
- the compressor screw (60) forms a functional unit with a shear sieve steel metal (70) placed in the inner space (62) of the housing (61) and extending in the longitudinal direction of the compressor screw for separating the disintegrated hygiene waste containing the solid components from the washing liquid with the constituents of the hygiene waste dissolved therein, whereby the shear sive steel metal (70) is made of a curved plate-shaped sieve body (70') with a partially circular vault, the radius of which corresponds to the outer radius of the compressor screw (60) and the vaulted sieve surface (70a) is placed lying without being spaced from the peripheral surface of the compressor screw (60) so that a shear-type combined action of the compressor endless screw (60) and the shear sieve steel metal (70) ensues,
- a drain (80) connected with the inner space (62) of the housing (61) with the compressor screw (60) with an integrated pump (81) for the washing liquid (WF) with the constituents of the hygiene waste dissolved therein is provided through which the washing liquid (WF) can be fed to a waste water duct (85), whereby the rest of the disintegrated hygiene waste which has solid constituents freed from the liquid can be fed to a collecting receiver (95) in the upper area (61b) of the housing (61) with the compressor screw (60),
- a compressor nozzle (100) with an upwards tapering section for separating rest liquid from the housing (61) is configured in the upper area (61 b) of the housing (61) with the compressor screw (60), this nozzle being connecting with the drain (80).

2. Device according to claim 1,
**characterized in that** the inner space (27) of the container (20) is connected with a cold water supply (28).

3. Device according to any of the claims 1 and 2,
**characterized in**
**that** the drum-type container (20) is made of an upper cylindrical container body (20a) which is converted on the bottom side into a tapering section (20b) which forms the transport screw (65).

4. Device according any of the claims 1 to 3,
**characterized in**
**that** the shear sieve sheet metal (70) is configured as an insert body which can be placed into the housing (61) with the compressor screw (60).

5. Device according to any of the claims 1 to 4,
**characterized in**
**that** a number of shearing knives (31) which is placed distributed over the disk surface is provided on the wall surface of the knives disk (30) which is turned to the inner space (27) of the container (20).

6. Device according to any of the claims 1 to 5,
**characterized in**
**that** the length of the container (20) corresponds approximately to the diameter of the circular front walls (21, 22) of the cylindrical container body (20a) of the container (20), whereby the diameter of the knives disk (30) corresponds approximately to the diameter of the front walls (21, 22) in the area above the transport screw (65) of the container (20).

7. Device according to any of the claims 1 to 6,
**characterized in**
**that** the container (20) is supported elastically in the frame (12) of the housing (11).

8. Device according to any of the claims 1 to 7,
**characterized in**
**that** each shearing knife (31) has an optimized knife polished surface for an effective disintegration of the hygiene waste and is made of an approximately rectangular fixing plate (150) and of a plate-shaped and approximately triangular knife body (155) placed upright on the fixing plate (150) and running diagonally with respect to the fixing plate, knife body the one side wall surface (156) of which is curved inwards arc-shaped, whereby the other side wall surface (157) is plane and has two side sections (158, 159) bent off to the side wall surface (157) as well as an upper bent-off section surface (160) situated in the upper tip area of the knife body (155) which is converted to a tapering cutting surface (162) by forming a cutting edge (161) with a knife-type reduced tearing edge (162), whereby the other side edge extending from the tip area of the knife body (155) to the side section surface (158) is configured as a cutting edge and cutting surface (13), whereby the reduced tearing edge (162) is configured as a two-sided polished taper.

9. Device according to any of the claims 1 to 8,
**characterized in**
**that** the container (20) is provided with a self-filling air trap (50) for aeration.

10. Method for carrying out an in-situ disposal of hygiene waste by using a device according to the claims 1 to 9,
**characterized in**
**that**
a) the hygiene waste is treated, torn and disintegrated in a drum-type container (20) by simultaneously supplying washing water, preferably cold washing water, chemicals, hygiene liquid and disinfectants by means of shearing knives (31) of a rotatably driven knives disk (30), then
b) the disintegrated product is fed to a compressor screw (60) for separating the disintegrated, solid component containing hygiene waste from the washing liquid by means of a shearing sieve sheet metal (70) with a simultaneous shear-type combined action of the compressor screw (60) with the shear sieve steel metal (70) for avoiding an obstruction of the shear sieve steel metal (70), then
c) the washing liquid with the soluble constituents of the hygiene waste solved therein is fed to a discharge duct and the rest of the hygiene waste freed from the washing liquid and having solid constituents to a collecting receiver (95).

11. Method according to claim 10,
**characterized in**
**that** chemicals for treating the superabsorbers such as solid products made of plastics or superabsorbing polymer products or gel bodies are fed for the treating process of the hygiene waste in the container (20).

## Revendications

1. Dispositif pour l'élimination sur place des déchets d'articles hygiéniques, les déchets d'articles hygiéniques pouvant être fragmentés mécaniquement au moyen du dispositif et pouvant être dissous au moins partiellement dans un liquide de lavage de telle manière que le liquide ainsi obtenu peut être séparé du reste des déchets d'articles hygiéniques résiduels fragmentés, le liquide peut être amené à un conduit d'évacuation et les déchets d'articles hygiéniques résiduels fragmentés pouvant être drainés ainsi qu'évacués séparément et le dispositif (10) étant constitué par un boîtier (11) dans lequel un récipient fixe de type tambour (20) est prévu avec un axe médian horizontal (MA) et avec un orifice de remplissage (25) pour les déchets d'articles hygiéniques ainsi qu'avec une conduite d'amenée (28) pour un liquide de lavage dosé (WF), une conduite d'amenée (29) pour un liquide hygiénique dosé (HF) pour supprimer les odeurs et pour la désinfection et une conduite d'amenée pour des produits chimiques dosés à l'état poudreux ou liquide pour traiter les superabsorbants, un dispositif d'évacuation pour l'évacuation des déchets d'articles hygiéniques fragmentés chargés de liquide de lavage à partir de la zone du fond du récipient qui est intégré au corps du récipient (20) étant prévu dans la zone du fond du récipient (20), un disque de couteaux (30) vertical, qui peut être entraîné en rotation, avec des couteaux déchireurs (31) dirigés vers l'espace intérieur (27) du récipient (20) pour arracher et fragmenter les déchets d'articles hygiéniques étant placé dans l'espace intérieur (27) du récipient (20) et une vis sans fin de compression qui suit le dispositif d'évacuation étant placée dans un boîtier à peu près tubulaire, le dispositif d'évacuation étant guidé jusque dans la zone côté fond de la vis sans fin de compression et un dispositif de commutation à programme ou un dispositif librement programmable étant prévu pour la commande de l'alimentation en liquide de lavage et des pompes de dosage (28', 29', 129') pour le liquide hygiénique et pour les produits chimiques, du dispositif de commande (35) pour le disque de couteaux (30) ainsi que du dispositif de commande (66) pour la vis sans fin de compression (60) et les pompes,
**caractérisé en ce**
**que**
- le dispositif d'évacuation (165) est configuré comme une vis sans fin de transport (65), la paroi latérale du récipient (23) étant convertie côté fond en une section s'effilant coniquement vers le bas qui débouche dans la vis sans fin de transport (65),
- la vis sans fin de compression (60) placée dans l'espace intérieur (62) du boîtier tubulaire (61) approximativement perpendiculaire suit la vis sans fin de transport (65), la vis sans fin de transport (65) étant guidée jusque dans la zone côté fond de la vis sans fin de compression (60) et la vis sans fin de compression (60) présentant un pas différent et une épaisseur de traverse différente,
- la vis sans fin de compression (60) forme une unité fonctionnelle avec une tôle de criblage cisaillante (70) placée dans l'espace intérieur (62) du boîtier (61) et qui s'étend dans le sens longitudinal de la vis sans fin de compression pour séparer les déchets d'articles hygiéniques fragmentés qui contiennent des composants solides du liquide de lavage avec les constituants des déchets d'articles hygiéniques dissous dans celui-ci, la tôle de criblage cisaillante (70) étant constituée par un corps de criblage (70') voûté en forme de plaque avec une voussure partiellement circulaire dont le rayon correspond au rayon extérieur de la vis sans fin de compression (60) et la surface de criblage voûtée (70a) étant placée à plat sans distance par rapport à la surface circonférentielle de la vis sans fin de compression (60) si bien qu'il se règle une action combinée de type cisaillement de la vis sans fin de compression (60) et de la tôle de criblage cisaillante (70),
- une conduite d'évacuation (80) reliée à l'espace intérieur (62) du boîtier (61) avec la vis sans fin de compression (60) étant prévue avec une pompe intégrée (81) pour le liquide de lavage (WF) avec les constituants des déchets d'articles hygiéniques qui y sont dissous, conduite par laquelle le liquide de lavage (WF) peut être amené à un canal d'eaux usées (85), le reste des déchets d'articles hygiéniques fragmentés et présentant des constituants solides, libéré du liquide, pouvant être amené à un récipient collecteur (95) dans la zone supérieure (61 b) du boîtier (61) avec la vis sans fin de compression (60),
- une tuyère de compresseur (100) avec une section s'effilant vers le haut étant configurée dans la zone supérieure (61 b) du boîtier (61) avec la vis sans fin de compression (100) pour séparer le liquide résiduel à partir du boîtier (61), tuyère qui est reliée à la conduite d'évacuation.

2. Dispositif selon la revendication 1,
**caractérisé en ce**
**que** l'espace intérieur (27) du récipient (20) est relié à une alimentation en eau froide (28).

3. Dispositif selon l'une des revendications 1 et 2,
**caractérisé en ce**
**que** le récipient de type tambour (20) est constitué par un corps de récipient cylindrique supérieur (20a) qui se convertit côté fond en une section s'effilant (20b) qui forme la vis sans fin de transport (65).

4. Dispositif selon l'une des revendications 1 à 3,
**caractérisé en ce**
**que** la tôle de criblage cisaillante (70) est configurée comme un corps d'insertion qui peut être placé dans le boîtier (61) avec la vis sans fin de compression (60).

5. Dispositif selon l'une des revendications 1 à 4,
**caractérisé en ce**
**qu'**un certain nombre de couteaux déchireurs (31) placé en étant réparti sur la surface du disque est prévu sur la surface de la paroi du disque de couteaux (30) qui est tournée vers l'espace intérieur (27) du récipient (20).

6. Dispositif selon l'une des revendications 1 à 5,
**caractérisé en ce**
**que** la longueur du récipient (20) correspond approximativement au diamètre des parois frontales circulaires (21, 22) du corps de récipient cylindrique (20a) du récipient (20), le diamètre du disque de couteaux (30) correspondant approximativement au diamètre des parois frontales (21, 22) dans la zone au-dessus de la vis sans fin de transport (65) du récipient (20).

7. Dispositif selon l'une des revendications 1 à 6,
**caractérisé en ce**
**que** le récipient (20) est positionné élastique dans le cadre (12) du boîtier (11).

8. Dispositif selon l'une des revendications 1 à 7,
**caractérisé en ce**
**que** chaque couteau déchireur (31) présente, pour une fragmentation efficace des déchets d'articles hygiéniques, un affûtage optimalisé et est constitué par une plaque de fixation approximativement rectangulaire (150) et par un corps de couteau en forme de plaque et approximativement triangulaire (155), placé perpendiculaire sur la plaque de fixation (150) et allant dans le sens diagonal par rapport à celle-ci, corps de couteau dont l'une des surfaces de parois latérales (156) est voûtée vers l'intérieur de forme arquée, l'autre surface de paroi latérale (157) étant plane et présentant deux sections latérales (158, 159) coudées vers la surface de paroi latérale (157) ainsi qu'une surface de section coudée supérieure (160), située dans la zone supérieure de la pointe du corps de couteau (155), qui se convertit en une surface de coupe (162) se terminant en pointe en configurant une arête de coupe (161) avec une arête d'arrachage (162) rentrée de type couteau, l'autre arête latérale qui s'étend de la zone de la pointe du corps de couteau (155) vers la surface de section latérale (158) étant configurée comme une arête de coupe et surface de coupe (163), l'arête d'arrachage (162) étant configurée comme un rétrécissement affûté des deux côtés.

9. Dispositif selon l'une des revendications 1 à 8,
**caractérisé en ce**
**que** le récipient (20) est pourvu d'un siphon inodore auto-remplissant (50) pour l'aération.

10. Procédé pour exécuter une élimination sur place de déchets d'articles hygiéniques en utilisant un dispositif selon les revendications 1 à 9,
**caractérisé en ce**
**que**
a) les déchets d'articles hygiéniques sont traités, déchirés et fragmentés dans un récipient de type tambour (20) tout en amenant simultanément de l'eau de lavage, de préférence de l'eau de lavage froide, des produits chimiques, du liquide hygiénique et des produits désinfectants au moyen de couteaux déchireurs (31) d'un disque de couteaux (30) entraîné en rotation, ensuite
b) le produit fragmenté est amené à une vis sans fin de compression (60) pour séparer les déchets d'articles hygiéniques contenant des composants solides fragmentés du liquide de lavage au moyen d'une tôle de criblage cisaillante (70) pour une action combinée simultanée de type cisaillante de la vis sans fin de compression (60) avec la tôle de criblage cisaillante (70) pour éviter une obstruction de la tôle de criblage cisaillante (70), ensuite
c) le liquide de lavage avec les constituants soluble des déchets d'articles hygiéniques qui y sont dissous est amené à un canal d'eaux usées et les résidus des déchets d'articles hygiéniques fragmentés et contenant des composants solides, libérés du liquide de lavage, étant amené à un récipient collecteur (95).

11. Procédé selon la revendication 10,
**caractérisé en ce**
**que** des produits chimiques pour le traitement des superabsorbants, tels que des produits solides en matière plastique ou des produits polymères superabsorbants ou des corps en gel, sont amenés pour le processus de traitement des déchets d'articles hygiéniques dans le récipient (20).
